# EUROPÄISCHE PATENTANMELDUNG

(11) **EP 0 874 043 A1**
(43) Veröffentlichungstag der Anmeldung: **28.10.1998**
(21) Anmeldenummer: 98102325.2
(22) Anmeldetag: 11.02.1998
(51) Int. Cl.: C12N 1/12

(54) **Verfahren zur Herstellung von Biomasse mittels Photosynthese**

(30) Priorität: 10.04.1997 DE 19714818
(71) Anmelder: Preussag AG, 30625 Hannover (DE)
(72) Erfinder: Broneske, Jürgen, Dr., 14943 Felgentreu (DE); Pulz, Otto, Dr., 14558 Bergholz-Rehbrücke (DE); Franke, Horst, 14558 Bergholz-Rehbrücke (DE); Döbel, Katrin, 14558 Bergholz-Rehbrücke (DE); Oehlmann, Hans-Ulrich, 31141 Hildesheim (DE); Uphoff, Rainer, Dr., 38122 Braunschweig (DE)
(74) Vertreter: Köckeritz, Günter

(57) **Zusammenfassung**

Die Erfindung betrifft ein Verfahren zur Herstellung von Biomasse mittels Photosynthese. Insbesondere betrifft die Erfindung ein Verfahren zur Kultivation von Mikroalgen und ermöglicht eine effektive Produktion von Biomasse durch Fixierung von Kohlendioxid aus CO₂-haltigen Abgasen.

Der vorliegenden Erfindung liegt die Aufgabe zugrunde, ein Verfahren zur Herstellung von Biomasse mittels Photosynthese, insbesondere zur Kultivation von Mikroalgen vorzustellen, welches eine effektive Produktion von Biomasse durch Fixierung von Kohlendioxid aus CO₂-haltigen Abgasen unter Vermeidung der Nachteile bekannter Verfahren ermöglicht. Mit der vorliegenden Erfindung sollen bekannte derartige Verfahren hinsichtlich ihrer Energiebilanz, ihres Herstellungsaufwandes und einer optimalen Ausnutzung der zur Verfügung stehenden Lichtenergie dahingehend verbessert werden, daß diese für einen großtechnischen Einsatz geeignet sind.
Erfindungsgemäß erfolgt die Herstellung von Biomasse mittels Photosynthese, wobei durch Beimpfen einer Nährlösung mit Mikroorganismen eine Kultursuspension hergestellt wird und die Kultursuspension mit einem CO₂-enthaltenden Gas unter Sättigung der im Gas enthaltenen Komponenten bis zur Blasenfreiheit konditioniert wird. Die Suspension wird anschließend unter Erzeugung eines gleichmäßigen Strömungsprofiles in einen Reaktor geleitet, in dem bei Belichtung und Einstellung einer optimalen Fließgeschwindigkeit der Lösung eine Abreicherung des in der Kultursuspension enthaltenen CO₂ durch Aufbau energiereicher Kohlenstoffverbindungen und Bildung weiterer Stoffwechselprodukte erfolgt.

## Beschreibung

Die Erfindung betrifft ein Verfahren zur Herstellung von Biomasse mittels Photosynthese. Insbesondere betrifft die Erfindung ein Verfahren zur Kultivation von Mikroalgen und ermöglicht eine effektive Produktion von Biomasse durch Fixierung von Kohlendioxid aus CO₂-haltigen Abgasen.

Mikroalgen lassen sich gut kultivieren, und daher sind Techniken zur Nutzung der hohen photosynthetischen Produktivität aus zahlreichen Patenten und Publikationen bekannt (Biotechnology and Bioengineering, Vol.35, 809, 1990, H.Gutermann). Häufig wird das Phytoplankton zur Gewinnung von Wertstoffen oder Pharmazeutika wie Polysacchariden, polyungesättigten Fettsäuren, Farbstoffen, Vitaminen usw. geerntet (Algae and Human Affairs, Cambridge University Press, Cambridge, 1988, K.G.Sprenger et al.). Aber auch als proteinreiche Futterstoffe finden die Biomassen aus Algen Verwendung.

Unter den Verfahren, die zur Erzeugung eines Massenproduktes angewendet werden, sind zunächst die häufig beschriebenen offenen Systeme zu nennen Hierunter werden raceway ponds, runde Becken und evt. natürliche Lagunen mit einer Wassertiefe von in der Regel 10 - 30 cm verstanden. In diesen Systemen werden die in Suspension vorliegenden Algen mit Hilfe von Rührvorrichtungen wie z. B. Schaufelrädern, Propellern oder Archimedischen Schrauben agitiert, um ein Absetzen der Mikroorganismen zu verhindern. Gleichzeitig gelangen alle Algen durch intensives Mischen in die oberen, von intensiverem Licht durchstrahlten Wasserschichten ( Applied Biochemistry and Biotechnology, Vol.51/52, 681, 1995, H. Matsumoto et al.).
Die Produktivität der Mikroalgen in offenen Systemen hängt stark von der Lichtintensität sowie der Fließgeschwindigkeit ab, die eng mit der Technik der Agitation verbunden ist. Typische Biomasseerträge in offenen Systemen liegen bei 8 - 12 g/m² d.

Eine weitere Steigerung der Biomasseerträge läßt sich durch Verfahren, in denen geschlossene Reaktoren eingesetzt werden, erzielen. Dort lassen sich die Kultivationsbedingungen wie Temperatur, Nährstoffangebot, CO₂-Angebot und Lichtintensität genauer als in offenen Systemen steuern bzw. kontrollieren. Bei den geschlossenen Systemen handelt es sich in der Regel um Platten- oder Röhrenreaktoren, die direkt mit Sonnenlicht bzw. künstlichem Licht bestrahlt werden oder in die das Licht durch Lichtleitsysteme. Spiegel und Linsen eingekoppelt wird (Advanves in Biochemical Engineering/Biotechnology, Vol.46, 63, 1992, I.Karube et al.). Die Biomasseerträge liegen abhängig von der Algenspezies sowie vom Kultivierungsverfahren in den genannten Systemen bei 15 - 25 g/m² d.

Da der Kohlenstoffgehalt der Biomasse ca. 60 Massen- % beträgt, wird deutlich, daß CO₂ während der Photosynthese den größten Beitrag zum Stoffaufbau liefert und somit nach preisgünstigen CO₂-Quellen gesucht werden muß Der CO₂-Gehalt der Atmosphäre ist zu gering, um die erforderlichen Produktivitäten zu gewährleisten. Rauchgas und andere industrielle Abgase haben dagegen das Potential, auch große Algenfarmen mit ausreichenden Mengen an CO₂ zu versorgen. Ein Screening nach Algenspezies, die hohe CO₂-Konzentrationen und die im Abgas enthaltenen Spurengase wie SOₓ, NOₓ und CO tolerieren, ist aus verschiedenen Druckschriften bekannt (Plant Physiol.,82,610, 1986, Y.Marcus et al.; Plant Physiol,91,514, 1989, G.D Price; Plant Physiol. 94,760, 1990, T.Ogewa). Untersuchungen über das Wachstumsverhalten während der Versorgung der Mikroalgen mit Abgasen zeigten, daß diverse marine Algen, z. B. Tetraselmis suecicca, Nannochloropsis/Phaeodactylum, durch eine Verwendung der Abgase nicht in ihrem Wachstum eingeschränkt wurden und die Kulturen für ein Jahr stabil blieben ( Applied Biochemistry and Biotechnology, Vol.51/52, 681, 1995, H. Matsumoto et al.). Diese Ergebnisse können jedoch nicht verallgemeinert werden und sind stark von der verwendeten Spezies und den Kultivationsbedingungen abhängig.

Weiterhin muß die Ernte der Biomasse auf die jeweiligen Mikroorganismen abgestimmt werden, da die entsprechenden Verfahren stark von der Erntekonzentration, der Organismengröße sowie den physiologischen Eigenschaften abhängig sind. Aus der Biotechnologie sowie der Abwassertechnologie sind eine Vielzahl von Verfahren zur Zellernte und Feststoffabtrennung bekannt. Beispielhaft seien Tellerzentrifuge, Lamellenseparator und Filtration mit Flockungsmitteln genannt ( 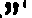 Micro - algal Technology", Cambridge University Press, 1988, M.A.Borowitzka, L.J.Borowitzka (eds.)).
Gleiches wie für die Ernte gilt auch für den Aufschluß sowie die Extraktion von Mikroalgen. Die zum Einsatz kommenden Verfahren müssen auf die Zelle und den Verwendungszweck der Biomasse abgestimmt werden.

Der vorliegenden Erfindung liegt die Aufgabe zugrunde, ein Verfahren zur Herstellung von Biomasse mittels Photosynthese, insbesondere zur Kultivation von Mikroalgen vorzustellen, welches eine effektive Produktion von Biomasse durch Fixierung von Kohlendioxid aus CO₂-haltigen Abgasen unter Vermeidung der Nachteile bekannter Verfahren ermöglicht. Mit der vorliegenden Erfindung sollen bekannte derartige Verfahren hinsichtlich ihrer Energiebilanz, ihres Herstellungsaufwandes und einer optimalen Ausnutzung der zur Verfügung stehenden Lichtenergie dahingehend verbessert werden, daß diese für einen großtechnischen Einsatz geeignet sind.

Erfindungsgemäß wird diese Aufgabe durch die Merkmale des Anspruchs 1 gelöst. Vorteilhafte Ausbildungen des Verfahrens sind in den Ansprüchen 2 bis 20 enthalten. Die Erfindung beinhaltet weiterhin die Verwendung besonders bevorzugter Algenspezies.

Nach der vorliegenden Erfindung erfolgt die Herstellung von Biomasse mittels Photosynthese, wobei durch Beimpfen einer Nährlösung mit Mikroorganismen eine Kultursuspension hergestellt wird und die Kultursuspension mit einem CO₂-enthaltenden Gas unter Sättigung der im Gas enthaltenen Komponenten bis zur Blasenfreiheit konditioniert wird. Die Suspension wird anschließend unter Erzeugung eines gleichmäßigen Strömungsprofiles in einen Reaktor geleitet, in dem bei Belichtung und Einstellung einer optimalen Fließgeschwindigkeit der Lösung eine Abreicherung des in der Kultursuspension enthaltenen CO₂ durch Aufbau energiereicher Kohlenstoffverbindungen und Bildung weiterer Stoffwechselprodukte erfolgt.

Mit der vorliegenden Erfindung wird ein Verfahren vorgestellt, welches sich gegenüber bislang bekannten Verfahren durch ein reduziertes Kontaminationsrisiko, reproduzierbare Kultivationsbedingungen, hohe Flexibilität in Bezug auf Umwelteinflüsse, geringeren Platsbedarf und reproduzierbare Biomassequalitäten auszeichnet.

Nach einem besonderen Merkmal der Erfindung handelt es sich bei dem Stoffwechselprodukt um ein O₂-haltiges Gas, welches durch Phasentrennung nach Durchgang durch den Reaktor abgetrennt wird und einer weiteren Verwendung zugeführt werden kann. Diese Sauerstoffproduktion macht das erfindungsgemäße Verfahren besonders effizient.

Nach einem bevorzugten Merkmal der Erfindung werden als Mikroorganismen phototrophe Organismen und/oder Zellkulturen, insbesondere Mikroalgen eingesetzt. Es wurde gefunden, daß mit den Mikroalgenspecies Chlorella vulgaris und/oder Scenedesmus spp./Microcystis (Mischkultur) sich besonders gut die oben beschriebenen Vorteile des Verfahrens erreichen lassen.

Nach einem weiteren Merkmal der Erfindung werden nach dem vorliegenden Verfahren Algenbiomasse und deren Stoffwechselprodukte erzeugt, wobei als Nährlösung Wasser und Stoffe, die für den Aufbau energiereicher Kohlenstoffverbindungen erforderlich sind, eingesetzt werden. Dabei handelt es sich um Stickstoffverbindungen, Phosphate, Kalium-, Calcium-, Magnesiumverbindungen und Spurenelemente. Nach einem anderen Merkmal der Erfindung enthält die Nährlösung auch organische Substanzen, z. B. Kohlenhydrate wie Zucker und/oder Acetate und/oder puffernde Substanzen wie Tris und/oder Borax.

Nach einem besonderen Merkmal der Erfindung kann die Nährlösung auch nährstoffhaltige Abwässer, einschließlich Klärschlämme und/oder synthetische Lösungen enthalten. Dadurch wird ein äußerst effektiver Einsatz derartiger Abwässer ermöglicht.

Ein ebenfalls bevorzugtes Merkmal der Erfindung beinhaltet, daß als CO₂-enthaltendes Gas ein Abgas, und zwar insbesondere ein Abgas aus Verbrennungsprozessen, Kalkbrennprozessen und /oder metallurgischen Prozessen eingesetzt wird. Damit müssen derartige Abgase nicht mehr an die Umwelt abgegeben werden, sondern können einer sinnvollen Verwendung zugeführt werden. Die Algenspezies erhalten darüberhinaus ausreichende Mengen an CO₂, um die erforderliche Produktivität zu gewährleisten.

Nach einem weiteren Merkmal der Erfindung erfolgt die Konditionierung der Kultursuspension mit dem CO₂-enthaltenden Gas unter Sättigung der im Gas enthaltenen Komponenten in einem Gaswäscher. Die Komponenten des Gases lösen sich dabei in der Kultursuspension bis zur Sättigung und Komponenten der Kultursuspension, deren Konzentration die Gaslöslichkeit in der Kultursuspension überschreitet, werden ausgetragen. Die Suspension verläßt den Gaswäscher blasenfrei. Mit dem Gaswäscher ist ein geregelter CO₂-Eintrag bei geringen CO₂-Verlusten möglich.

Ein bevorzugtes Merkmal der Erfindung beinhaltet, daß das aus Verbrennungsprezessen stammende CO₂-enthaltende Abgas in einem Abgaskühler temperiert wird und das dabei entstehende Kondensat mit dem CO₂-enthaltende Abgas über einen Gaswäscher in den Reaktor geleitet wird. Das Kondensat wird damit Bestandteil der Kultursuspension und einer sinnvollen Verwendung zugeführt .

Nach einem besonders bevorzugten Merkmal der Erfindung wird das gleichmäßige Strömungsprofil der konditionierten Kultursuspension dadurch erzeugt, daß diese in einen Reaktor eingeleitet wird, der aus mindestens einem einstückig extrudierten transparenten Plattenmodul besteht, welches eine Vielzahl von übereinanderliegenden und parallel zueinander verlaufenden durchgehenden Kanälen aufweist, deren Eintritts- und Austrittsseiten in einen Flüssigkeitssammler und/oder Flüssigkeitsverteiler münden, wobei der Flüssigkeitsverteiler in Fließrichtung eine Querschnittsreduzierung und der Flüssigkeitssammler eine Querschnittserweiterung aufweist. Dadurch kann erfindungsgemäß über alle Kanäle eine optimale, gleichmäßige Fließgeschwindigkeit der Lösung im Reaktor eingestellt werden. Es wird mit überraschend einfachen Mitteln erreicht, daß mit geringen Strömungsgeschwindigkeiten turbulente Verhältnisse im Kulturmedium erhalten werden, die für eine optimale Lichtenergieausnutzung erforderlich sind. Darüberhinaus kann der Strömungswiderstand im Vergleich zu den bekannten Lösungen erheblich verringert werden.

Letztendlich wird dadurch die erforderliche Pumpleistung wesentlich reduziert, wodurch die Energiebilanz besonders vorteilhaft verbessert wird. Die Verbindung mehrerer Plattenmodule ermöglicht außerdem den Aufbau beliebig großer Reaktoren für den großtechnischen Einsatz aus einfachen Standardbauteilen.

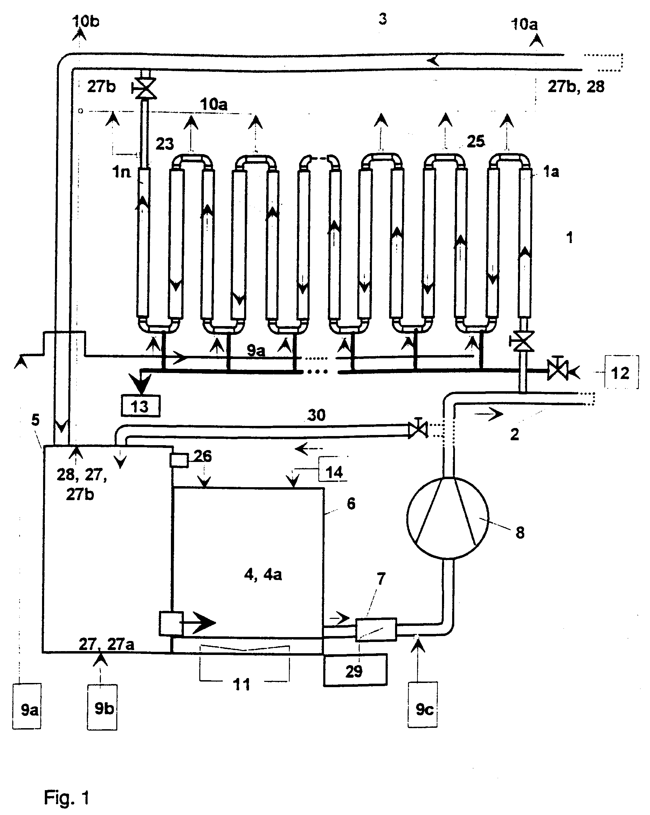

Nachfolgend soll die Erfindung anhand eines Ausführungsbeispieles näher erläutert werden.
In der Abbildung zeigt die
- Figur 1:: eine beispielhafte Ausführungsform des Reaktors und den schematischen Ablauf des Verfahrens.

Aus dem Brennprozeß eines Kalkbrennofens wird ein Abgas 27a mit einer CO₂-Konzentration von ca. 25 % einem Abgaskühler (nicht dargestellt) zugeführt. Im Abgaskühler wird das CO₂-enthaltende Abgas auf eine Temperatur von ca. 35 °C temperiert. Während des Kühlprozesses entsteht ein Kondensat.
Das CO₂-enthaltende Abgas und das Kondensat werden über einen Gaswäscher 5 in einen Reaktor geleitet, wobei im Gaswäscher 5 eine Kultursuspension 4a mit dem CO₂-enthaltenden Abgas 27a und dem Kondensat (nicht dargestellt) unter Sättigung der im Abgas enthaltenen Komponenten bis zur Blasenfreiheit konditioniert wird. Die Kultursuspension 4a besteht aus einer Nährlösung und einer Mikroalgenspecies Chlorella vulgaris. Die Nährlösung besteht aus Wasser 9, anorganischen Nährstoffen und Tris(hydroxymethyl)-aminomethan.
Das Wasser wird dabei als Abwasser aus dem Kalkbrennofen zur Verfügung gestellt. Im Abwasser sind in geringer Konzentration anorganische Nährstoffe gelöst. Die Nährlösung (NL) beinhaltet dabei folgende anorganische Nährstoffe.

| **Komponente** | **Einsatzmenge ( g/l )** |
|---|---|
| | |

| *Makrobestendteile* | |
|---|---|
| Harnstoff | 0,3 |
| Ammoniumnitrat | 0,4 |
| Kaliumdihydrogenphosphat | 0,34 |
| Magnesiumsulfat | 0,5 |
| Eisensulfat | 0,5 |

| *Mikrobestandteile I* *pro Liter Stammlösung ( 0,01 ml/l NL )* | |
|---|---|
| Zinksulfat | 74,0 |
| Borax | 5,7 |
| Kobaltsulfat | 23,8 |
| Kupfersulfat | 23,6 |
| Mangansulfat | 410 |

| *Mikrobestandteile II* *pro Liter Stammlösung ( 0.01 ml/l NL )* | |
|---|---|
| Ammoniummolybdat | 9,2 |

Tris(hydroxymethyl)-aminomethan ist eine organische Substanz, die als Säurepuffer der Nährlösung in einer Konzentration von ca. 1,0 g/l zugesetzt wird.

Die konditionierte Kultursuspension zirkuliert im Reaktor welcher gemäß der Figur 1 aus einer Plattenmodulanlage 1 aus einstückig extrudierten transparenten Plattenmodul 1a...1n. einem Ausgleichsbehälter 6, einer Systempumpe 8, einer Meßstrecke 7, einem Vorlauf 2, einem Rücklauf 3 und dem Gaswäscher 5 besteht und wird mit Hilfe der natürlichen Sonneneinstrahlung 12 belichtet. Die konditionierte Kultursuspension gelangt aus dem Ausgleichsbehälter 6 durch die Systempumpe 8 über den Vorlauf 2 in das Plattenmodul 1a.
Das Plattenmodul 1a weist eine Vielzahl von übereinanderliegenden und parallel zueinander laufenden durchgehenden Kanälen auf. deren Eintritts- und Austrittsseiten in einem Flüssigkeitssammler 21 bzw. Flüssigkeitsverteiler 15 münden.

Der Flüssigkeitsverteiler 15 besitzt dabei in Fließrichtung eine Querschnittsreduzierung 19 und der Flüssigkeitssammier 21 besitzt eine Querschnittserweiterung 22.
Dadurch wird im Plattenmodul ein gleichmäßiges Strömungsprofil der konditionierten Kultursuspension gewährleistet.
Im Plattenmodul erfolgt das Biomassewachstum, die Abreicherung des gelösten CO₂ und die Anreicherung von Sauerstoff 28.
Nach dem Plattenmodul gelangt die Kultursuspension 4a über den Rücklauf 3 und den Gaswäscher 5 in den Ausgleichsbehälter 6 zurück.

Das CO₂-enthaltende Abgas 27a wird mit Hilfe des Gaswäschers 5 in der Kultursuspension 4a gelöst, und der im Verlauf der photobiologischen Prozesse gebildete Sauerstoff 28 mit den nicht genutzten Komponenten des Abgases 27b wird ausgetragen.

Die Konditionierung des CO₂-enthaltende Abgases erfolgt in Abhängigkeit der pH - und Temperatur- und Optischen Dichtewerten der Kultursuspension, welche in der Meßstrecke 7 vor der Systempumpe 8 erfaßt werden.
Die Messung, Steuerung, Regelung und Speicherung von Zustandsgrößen der Kultursuspension und des CO₂-enthaltenden Abgases erfolgt dabei über eine zentrale Meß-, Steuer-, Regel- und Speichereinheit 29.

Die Trockenmassekonzentration (TM) der Mikroalgenspecies Chlorella vulgaris in der Kultursuspension beträgt ca. 2 g Trockensubstanz/l. Im Verlaufe des Wachstums der Biomasse werden energiereiche Kohlenstoffverbindungen aufgebaut. Die Biomasseernte erfolgt bei einer Trockenmassekonzentration in der Kultursuspension von > 2 g/l.
Mit Hilfe der Systempumpe 8 wird die Kultursuspension einer Ernteeinrichtung (nicht dargestellt) zugeführt.
Die Erntevorrichtung besteht aus einem Zwischenspeicher, einer Förderpumpe und einer Zentrifuge. Die Zentrifuge trennt die Biomasse von der Nährlösung, welche wieder in den Reaktor zurückgeführt wird. Die Biomasse besitzt eine Trockenmassekonzentration von ca. 120 g/l und ist dementsprechand feststoffreich, aber pumpfähig.

Die somit erhaltene Biomasse wird einer Verarbeitungseinrichtung (nicht dargestellt) zugeführt. Diese Verarbeitungseinrichtung ist eine Einrichtung, die auf extraktiver Basis arbeitet. Die Biomasse wird einer Lösungsmittelextraktion unterzogen, wobei die energiereichen Kohlenstoffverbindungen als Energieträger gewonnen werden.

In einem weiteren Verfahrensschritt (nicht dargestellt) werden die gewonnen energiereichen Kohlenstoffverbindungen einer Veresterung unterworfen und somit ein Biodiesel hergestellt. Bis zum Zeitpunkt des Verbrauchs wird der Biodiesel in einem Speicher zwischengelagert.

## Patentansprüche

1. Verfahren zur Herstellung von Biomasse mittels Photosynthese, wobei durch Beimpfen einer Nährlösung mit Mikroorganismen eine Kultursuspension hergestellt wird,
- die Kultursuspension mit einem CO₂-enthaltenden Gas unter Sättigung der im Gas enthaltenen Komponenten bis zur Blasenfreiheit konditioniert wird,
- die Suspension unter Erzeugung eines gleichmäßigen Strömungsprofiles in einen Reaktor geleitet wird, in dem bei Belichtung und Einstellung einer optimalen Fließgeschwindigkeit eine Abreicherung des in der Kultursuspension enthaltenen CO₂ durch Aufbau energiereicher Kohlenstoffverbindungen und Bildung weiterer Stoffwechselprodukte erfolgt.

2. Verfahren nach Anspruch 1,
dadurch gekennzeichnet, daß das Stoffwechselprodukt ein O₂-haltiges Gas ist, welches durch Phasentrennung nach Durchgang durch den Reaktor abgetrennt wird und der weiteren Verwendung zugeführt wird.

3. Verfahren nach einem der obigen Ansprüche,
dadurch gekennzeichnet, daß
die im Reaktor erzeugte Biomasse aus der Kultursuspension abgetrennt und zu einer feststoffreichen, aber noch pumpfähigen Wasser/Biomasse-Suspension aufbereitet wird.

4. Verfahren nach Anspruch 3,
dadurch gekennzeichnet, daß
die Abtrennung der Biomasse unter Ausnutzung von Dichteunterschieden in Absetzbecken und/oder Zentrifugen und/oder unter Ausnutzung der Teilchengröße in Filtereinrichtungen erfolgt und die Kulturlösung in das Reaktorsystem zurückgeführt wird.

5. Verfahren nach einem der obigen Ansprüche,
dadurch gekennzeichnet, daß
Algenbiomasse und deren Stoffwechselprodukte erzeugt werden und als Nährlösung Wasser und Stoffe, die für den Aufbau energiereicher Kohlenstoffverbindungen erforderlich sind, eingesetzt werden.

6. Verfahren nach Anspruch 5,
dadurch gekennzeichnet, daß
die Nährlösung Stickstoffverbindungen, Phosphate, Kalium-, Calcium-, Magnesiumverbindungen und Spurenelemente enthält.

7. Verfahren nach den Ansprüchen 5 und 6,
dadurch gekennzeichnet, daß
die Nährlösung organische Substanzen, z. B. Kohlenhydrate wie Zucker und/oder Acetate und/oder puffernde Substanzen wie Tris(hydroxymethyl)aminomethan) und/oder Borax enthält.

8. Verfahren nach den Ansprüchen 5 bis 7,
dadurch gekennzeichnet, daß
als Nährlösung nährstoffhaltige Abwässer, einschließlich Klärschlämme und/oder synthetische Lösungen verwendet werden.

9. Verfahren nach einem der obigen Ansprüche,
dadurch gekennzeichnet, daß
als Mikroorganismen phototrophe Organismen und/oder Zellkulturen eingesetzt werden.

10. Verfahren nach Anspruch 9,
dadurch gekennzeichnet, daß
als Mikroorganismen Mikroalgen eingesetzt werden.

11. Verfahren nach den Ansprüchen 9 und 10,
dadurch gekennzeichnet, daß
als Mikroorganismen die Mikroalgenspecies Chlorella vulgaris und/oder Scenedesmus spp./Microcystis (Mischkultur) eingesetzt werden.

12. Verfahren nach einem der obigen Ansprüche,
dadurch gekennzeichnet, daß
als CO₂-enthaltendes Gas ein Abgas eingesetzt wird.

13. Verfahren nach einem der obigen Ansprüche,
dadurch gekennzeichnet, daß
als CO₂-enthaltendes Gas ein Abgas aus Verbrennungsprozessen, Kalkbrennprozessen und /oder metallurgischen Prozessen eingesetzt wird.

14. Verfahren nach einem der obigen Ansprüche,
dadurch gekennzeichnet, daß
die Konditionierung der Kultursuspension mit dem CO₂-enthaltenden Gas im Gaswäscher erfolgt, wobei sich die Komponenten des Gases in der Suspension bis zur Sättigung lösen und eine blasenfreie Kultursuspension erzeugt wird.

15. Verfahren nach einem der obigen Ansprüche,
dadurch gekennzeichnet, daß
das CO₂-enthaltende Abgas in einem Abgaskühler temperiert wird und das dabei entstehende Kondensat mit dem CO₂-enthaltenden Abgas über einen Gaswäscher in den Reaktor geleitet wird.

16. Verfahren nach Anspruch 15,
dadurch gekennzeichnet, daß
das Gas im Gegenstrom zur Kultursuspension durch den Gaswäscher geführt wird.

17. Verfahren nach einem der obigen Ansprüche,
dadurch gekennzeichnet, daß
das gleichmäßige Strömungsprofil der konditionierten Kultursuspension erzeugt wird, indem diese in einen Reaktor eingeleitet wird, der aus mindestens einem einstückig extrudierten transparenten Plattenmodul besteht, welches eine Vielzahl von übereinanderliegenden und parallel zueinander verlaufenden durchgehenden Kanälen aufweist, deren Eintritts- und Austrittsseiten in einen Flüssigkeitssammler und/oder Flüssigkeitsverteiler münden, wobei der in Fließrichtung gesehene Flüssigkeitsverteiler eine Querschnittsreduzierung und der Flüssigkeitssammler eine Querschnittserweiterung aufweist, wodurch über alle Kanäle eine optimale, gleichmäßige Fließgeschwindigkeit der Suspension im Reaktor eingestellt wird.

18. Verfahren nach einem der obigen Ansprüche,
dadurch gekennzeichnet, daß
die Belichtung der im Reaktor befindlichen Kultursuspension durch natürliche direkte und/oder diffuse Sonneneinstrahlung und/oder künstliche Beleuchtung erfolgt.

19. Verfahren nach einem der obigen Ansprüche,
dadurch gekennzeichnet, daß
die Biomasse zu einem Energieträger weiterverarbeitet wird.

20. Verfahren nach einem der obigen Ansprüche,
dadurch gekennzeichnet, daß
die Biomasse zur Produktion von chemischen und/oder pharmazeutischen Grund- und Wirkstoffen weiterverarbeitet wird.

21. Verwendung der Mikroalge Chlorella vulgaris mit der Fähigkeit zur Assimilation von Kohlendioxid zur Herstellung von Biomasse.

22. Verwendung der Mikroalgenmischkultur Scenedesmus spp./Microcystis mit der Fähigkeit zur Assimilation von Kohlendioxid zur Herstellung von Biomasse.
